# EUROPEAN PATENT APPLICATION

(11) **EP 1 146 433 A1**
(43) Date of publication of application: **17.10.2001**
(21) Application number: 00401059.1
(22) Date of filing: 14.04.2000
(51) Int. Cl.: G06F 17/00, G06F 19/00, A61B 5/04

(54) **Method and apparatus for detecting sleep disorders using the variability of the RR interval in an ECG waveform**

(71) Applicant: Novacor, 92500 Rueil-Malmaison (FR)
(72) Inventor: Antoniadis, Anestis, 92500 Rueil Malmaison (FR); Barthelemy, Jean-Claude, 92500 Rueil Malmaison (FR); Costes, Frédéric, 92500 Rueil Malmaison (FR); Duverney, David, 92500 Rueil Malmaison (FR); Gaspoz, Jean-Michel, 92500 Rueil Malmaison (FR); Lacour, Jean-René, 92500 Rueil Malmaison (FR); Pichot, Vincent, 92500 Rueil Malmaison (FR); Roche, Frédéric, 92500 Rueil Malmaison (FR)
(74) Representative: Moutard, Pascal Jean

(57) **Abstract**

The invention concerns a method for processing an RR intervals signal, derived from an ECG signal, comprising :
- storing RR intervals and calculating the variability of the RR intervals,
- calculating the power spectral density of the interbeat interval increment and exhibiting said portion of the spectrum thus obtained which is comprised below 0.04 Hz, or calculating the relative importance of the component of the spectrum which is comprised below 0.04 Hz over the total power spectral density and comparing said importance with a predetermined threshold value.

## Description

### Technical field and related art

The invention concerns the field of sleep apnea detection.

Obstructive sleep apnea syndrome (OSAS) is a growing health concern affecting up to 10% of middle-aged men. Today, its diagnosis depends on polysomnography recordings, which require the detection of various physiological signals, like airflow, oxygen saturation, heart rate and is usually made in a complex in-hospital technological medical environment. In other words, the prevalence of obstructive sleep apnea syndrome (OSAS) is high in developed countries but its diagnosis is costly. In spite of the high cardiovascular morbidity and mortality associated with this syndrome, the substantial inconvenience and cost of polysomnography recordings may delay routine evaluation. The introduction of more simple screening tools could help recognize patients with high probabilities of OSAS and, thus, focus on candidates likely to be afflicted with this disorder. So far, of the parameters recorded on an ambulatory basis and compared to the yield of complete polysomnography, only pulse oximetry showed results consistant with the severity of the disease (see for example : Stoohs R and Guilleminault C. MESAM 4: an ambulatory device for the detection of patients at risk for obstructive sleep apnea syndrome (OSAS). Sleep. 1992;101:1221-1227).

Oxymetry is not very convenient because it also has to be carried out in a medical environment, in a hospital. An oximeter (actually : an Infra-red light source) is attached to an individual's finger and provides an indication of oxygen saturation level. There is also the problem of constantly maintaining the oxymeter attached to the patient; it can sometimes detach itself or fall off the patient's finger.

In addition, methods for detecting sleep apnea all require a precise identification of the beginning and of the end of the sleep periods of the patient. Data are recorded only during these periods, which is very inconvenient.

There is thus the problem of finding a new method for detecting sleep apnea and new devices for data acquisition and for data processing for such a method.

There is also a need to find a method and a device for helping in the detection of sleep apnea without polysomnography recordings and on another basis than oximetry.

There is also a need for a cheap method for detecting sleep apnea, without any need for the patient to remain in an hospital environment.

There is also a further need for a method which can be carried out during the patient's sleep, but also before and after the patient's sleep.

### Summary of the invention

According to the ihe invention, sleep apnea syndrome is detected by measuring, sampling and processing heart rate signal only, without any further detection of any other signal, like oxygen saturation or airflow, and without any polysomnography recordings.

In a first aspect of the invention, quantification using time domain analysis of HRV (nocturnal cyclical heart rate variations), which already demonstrated its usefulness in other clinical fields, is shown to yield more pertinent diagnostic information regarding sleep disorder and in particular sleep apnea.

The sensitivity and the specificity of this seemingly simple tool was evaluated in patients for OSAS diagnosis. HRV variables were correlated with diseased status assessed with complete polysomnography to establish pertinent HRV diagnosis thresholds in one group of patients; then, the selected HRV thresholds were applied to comparable patients in order to validate the results.

Thus the invention concerns a method for processing an RR signal, derived from the normal beats of ECG signal, comprising :
- storing said RR intervals,
- calculating at least one of the following parameters:
   * difference between night and day square root of the mean of the sum of the squares of differences between adjacent normal RR intervals (Δ[D/N] r-MSSD),
   * difference between night and day mean of the standard deviation of all normal-normal intervals for all consecutive at least 5 minutes segments of the recording (Δ[D/N] SDNN index),
   * difference between night and daystandard deviation of the normal-normal intervals (Δ[D/N] SDNN),
- comparing said at least one parameter with at least one predetermined threshold value.

Parameters (Δ[D/N] r- MSSD), (Δ[D/N] SDNN index), (Δ[D/N] SDNN), are predictors for sleep apnea disorders, but are independant from each other.

The calculation of these parameters and their comparison with a predetermined threshold value comprises many advantages over the prior art methods. In particular, it does not require to maintain the patient in an hospital environment since recording of an ECG can be made with the help of a portable device, for example of the Holter type.

The at least one threshold value can be adjusted according to the required sensitivity and specificity.

The invention also concerns a device for processing an RR signal, derived from the normal beats of an ECG signal, comprising :
- means for storing RR intervals,
- means, or means specially programmed, for calculating at least one of the following parameters:
   * difference between night and day square root of the mean of the sum of the squares of differences between adjacent normal RR intervals (Δ[D/N] r-MSSD),
   * difference between night and day mean of the standard deviation of all normal-normal intervals for all consecutive at least 5 minutes segments of the recording (Δ[D/N] SDNN index),
   * difference between night and daystandard deviation of the normal-normal intervals (Δ[D/N] SDNN),
- means, or means specially programmed, for comparing said at least one parameter with at least one predetermined threshold value.

In another aspect of the invention, power spectral densisty of the interbeat interval increment was shown to be accurately predictive of sleep-related breathing disorders and an even more powerful diagnostic tool parameter.

Thus the invention also concerns a method for processing an RR intervals signal, derived from an ECG signal, comprising :
- storing RR intervals and calculating the variability of the RR intervals,
- calculating the power spectral density of the interbeat interval increment and exhibiting said portion of the spectrum thus obtained which is comprised below 0.04 Hz, or calculating the relative importance or the proportion of the component of the spectrum which is comprised below 0.04 Hz (or comprised between 0.01 Hz and 0.04Hz) over the total power spectral density.

This method can even be performed on ECG recordings made over a period longer than the patient's sleep, for example over 24 hours.

According to the invention, the interbeat interval increment spectral parameters is shown to be predictive for OSAS.

The invention also concerns a device for processing an RR intervals signal, derived from an ECG signal, comprising :
- means for storing RR intervals and the variability of the RR intervals,
- means, or means specially programmed, for calculating the power spectral density of the interbeat interval increment and exhibiting said portion of the spectrum thus obtained which is comprised below 0.04 Hz, and/or for calculating the relative importance of the component of the spectrum which is comprised below 0.04 Hz over the total power spectral density and comparing said importance with a predetermined threshold value.

Sleep-related breathing disorders are not only common today but they bear significant health consequences; since they may imply severe daytime repercussions, as well as increased cardiac morbidity. In spite of these complications, recent studies have reported that obstructive sleep apnea syndrome (OSAS), by far the more frequent of these disorders, was largely underdiagnosed in the middle-aged population, its prevalence reaching 10%.

The present invention offers a very precise and powerfull tool for identifying such sleep disorders, and is at the same time a very cheap way of making such an identification.

Because of limited financial resources for healthcare, it would be very cost-effective to establish OSAS diagnosis on the basis of the present invention.

It also offers a possibility to perform the follow-up of such sleep disorders, using ambulatory techniques instead of in-hospital full night monitoring.

### Brief description of the drawings

The various objects, advantages and novel features of the invention will be more fully apparent from a reading of the following detailed description in conjunction with the accompanying drawings, given for exemplary purposes and in which:
- figure 1 is a receiver operating characteristic (ROC) curve analysis for identification of OSAS of the very-low frequency component of the interbeat interval increment expressed as the ratio (%VLFI) of the VLFIpsd on the total spectral density.
- figure 2A is a linear correlation between the respiratory disturbance index (RDI) and the power spectral density of the very-low frequency (VLFIpsd) component of the interbeat interval increment.
- figure 2B shows the linear correlation between the respiratory disturbance index (RDI) and the very-low frequency component of HRV expressed as the ratio (%VLFI) of the VLFIpsd on the total spectral density.
- figure 3 shows the evolution of the interbeat interval increment expressed as the ratio (%VLFI) of the VLFIpsd on the total spectral density (VLFI%) observed after a 3-month CPAP treatment in 18 patients.
- figure 4 is part of and electrocardiogram, with two QRS signals,
- figure 5 shows a Holter representation over 24 hours.
- figure 6 shows steps of a method for creating an RR signal.
- figure 7 represents a patient with electrodes and an ECG recorder.
- figure 8 shows a recording device, together with a flash memory.
- figures 9 and 10 show an apparatus for processing an RR signal according to the invention.
- figure 11 is a partial view of the power spectral density of the interbeat interval increment for a normal patient,
- figure 12 is a partial view of the power spectral density of the interbeat interval increment for a patient showing apnea.
- figure 13 is a partial view of the power spectral density of the interbeat interval increment for a patient having apnea controlled with a CPAP treatment.

### Detailed description of preferred embodiments

The invention will first be described on the basis of two examples (I and II). A detailed description of a device according to the invention will then be given.

### EXAMPLE I

### a). Study groups

The population under study consisted of patients referred to hospital for a polysomnography recording because of clinically suspected obstructive sleep apnea syndrome (OSAS). The first group (G1), or derivation set, consisted of 91 patients (71 males, 20 females) with a mean (± SD) age of a 55.4±10.9 years (range, 30 to 78); the second group (G2), or validation set, consisted of 52 patients (34 males, 18 females) with a mean (± SD) age of a 52.7±12.1 years (range, 22 to 77). Exclusion criteria were permanent or paroxysmal atrial fibrillation, or permanent pacemaker. All patients underwent both a full polysomnography recording and ECG Holter monitoring.

### b). Sleep study and polysomnography scoring

Patients of both groups underwent nocturnal polysomnography recording using standard methods (see Rechtschaffen A and Kales A eds. A manual of standardized terminology, techniques and scoring systems for sleep stages of human subjects. Los Angeles: BIS/BRI UCLA; see also Guilleminault C. Sleep and Breathing. In: Guilleminault C, ed. Sleeping and Waking Disorders: Indications and Techniques. Stoneham, UK: Butterworth Publishers; 1982:155-182). The presence and the stages of sleep were monitored using two pairs of electroencephalographic leads (EEG: C₄-A₁, O₂-A₁), two pairs of electro-oculographic leads (EOC) and chin electromyographic (EMG) leads. Airflow was measured by an oronasal thermocoupler. Respiratory efforts were monitored using respiratory plethysmography with transducers placed around the chest and the abdomen. Arterial oxygen saturation was continuously recorded by pulse oximetry (with a device from Criticare Systems Inc., Waukesha, WI) during the whole night period.

The polysomnogram was scored manually according to standard criteria (see Thorpy MJ, and the Diagnostic Classification Steering Committee. The International Classification of Sleep Disorders : Diagnostic and Coding Manuel. Rochester, Minn: American Sleep Disorders Association; 1990:52-58).

Apnea was defined as the absence of airflow for more than 10 seconds in the presence of persistant respiratory efforts. Hypopnea was defined as the association of a reduction of 50% or more of the amplitude of respiratory efforts during at least 10 seconds, and a fall in arterial oxyhemoglobin saturation of at least 4%. The apnea/hypopnea index (AHI) was defined as the number of episodes of apnea and hypopnea per hour of sleep; the threshold to accept the diagnosis of OSAS was chosen as an AHI equal to or greater than 10; patients were thus classified as diseased (OSAS+) or non-diseased (OSAS-). Each episode of apnea was characterized by measuring apnea duration, mean and minimal arterial oxyhemoglobin saturation. Total sleep time (TST), number and duration of REM periods, number and duration of arousals, were measured. Total duration of arterial desaturation was quantitated as the cumulated time with arterial oxyhemoglobin saturation below 90%.

### c). ECG Holter monitoring and heart rate variability analysis

The Holter tapes were analyzed on a StrataScan 563 (Del Mar, Irvine, CA), equipped with a heart rate variability module. All variables were calculated for the 24-hour, the day-time (02:30 to 09:30 PM), and the night-time (00:00 to 07:00 AM) periods, and the differences (Δ[D/N]) between day-time and night-time values were computed as well.

HRV was evaluated using time domain analysis. To perform the analysis, each QRS complex was identified and the length between each QRS (RR interval) calculated. Only normal QRS are selected, ventricular and supraventricular QRS signals being discarded. Only normal to normal (NN) intervals were considered for the analysis. Two intervals separing successive normal beats are NN intervals if the second interval differs from the first one of less than 20%.

Several parameters describing the differences between RR intervals were calculated:
- the square root of the mean of the sum of the squares of differences between adjacent normal RR intervals (r-MSSD)
- the standard deviation of NN intervals (SDNN)
- the standard deviation of the averages of NN intervals in all (at least) 5-minute segments of the recording (SDANN)
- and the mean of the standard deviations of all NN intervals for the all consecutive (at least) 5-minute segments of the recording (SDNN Index).

### d). Statistical analysis

Data were analyzed with Statview and JMP (SAS Institute) softwares. Differences were considered as significant when p<0.05. Values were expressed as mean and 95% standard deviations (mean±SD).

In group 1 (derivation set), statistical analyses were performed to evaluate the ability of each variable to discriminate between diseased and non-diseased status. Thus, the dependent variable was diseased status (OSAS +/-). The independent variables analyzed were age, gender, body mass index (BMI) and, for HRV variables, their day and night values as well as the differences between their night and day values (Δ[D/N]), as night mean HR, Δ[D/N] mean HR, night r-MSSD, Δ[D/N] r-MSSD, night SDNN, ΔD/N] SDNN, night SDNN Index, Δ[D/N] SDNN Index, night SDANN, and Δ[D/N] SDANN.

ROC curve analysis was used (see Hanley JA, McNeil BJ. The meaning and use of the area under receiver operating characteristic (ROC) curve. Radiology. 1982; 143:29-36) with calculation of the areas (W) under the curves. A W value of 0.5 means that the variable distributions are similar in both populations; conversely, a W value of 1.0 means that the two populations variable distributions do not overlap at all.

Logistic regression models were also used to analyze the data via a simple logistic regression of the disease status versus each of the covariates to confirm the previously performed ROC curve analysis. Multiple logistic regression analysis was then used to evaluate the strength of the association of each variable with diseased status, after adjustment for the other variables. Also, the odds ratio were calculated individually for the same most significant independent variables.

Finally, a threshold value was determined for the most significant variables so as to obtain the better separation power between diseased and non-diseased status. Determination of the threshold values was based on the classification and regression tree (CART) methodology, (see Breiman L, Friedman JH, Olshen RA, Stone CJ. Classification and Regression Trees. Wadsworth International Group, Belmont, California 1994) which allows to split a continuous variable into two groups. All possible values were considered and the chosen value was that which maximized the Gini impurity criterion (see Clopper CJ, Pearson ES. The use of confidence or fiducial limits illustrated in the case of the binomial. Biometrika. 1934;26:404).

Sensitivity and specificity were also estimated against the diseased and non-diseased status, with their simultaneous 'exact' 95% confidence interval, i.e. the v95% confidence interval for sensitivity and specificity.

In group 2 (validation set), the sensitivity and the specificity were calculated for diseased and non-diseased status using two of the three most significant independent variables identified by CART methodology in group 1.

### e). Results

In the derivation set (Group 1), the diagnosis of OSAS was established in 39 (42.8%) out of the 91 patients using polysomnography recording. The clinical and the time domain HRV variables of OSAS+ and OSAS- patients are summarized in Table 1.

**TABLE 1.**

| Clinical and Heart Rate Variability (HRV) Characteristics in the derivation set (Group 1) with (OSAS+) or without (OSAS-) Obstructive Sleep Apnea Syndrome | | | |
|---|---|---|---|
| Clinical characteristics | OSAS + | OSAS - | p |
| | (n = 39) | (n = 52) | |
| Age, y (mean±SD) | 55.9±12.0 | 54.9±10.0 | ns |
| Male, n (%) | 31(79) | 41(79) | ns |
| BMI, kg/m² (mean±SD) | 28.7±9.6 | 27.9±7.9 | ns |
| Diabetes, n (%) | 6 (15) | 6 (11.5) | ns |
| Hypertension, n (%) | 14 (36) | 15 (29) | ns |
| MI, n (%) | 1 (2.6) | 3 (5.8) | ns |
| CHF, n (%) | 4 (10) | 3 (5.8) | ns |
| Note: BMI, body mass index; MI, old myocardial infarction; CHF, cardiac heart failure; OSAS+, patients with obstructive sleep apnea syndrome; OSAS-, patients whitout obstructive sleep apnea syndrome. | | | |

| HRV characteristics | OSAS + | OSAS - | p |
|---|---|---|---|
| Day SDNN, ms (mean±SD) | 80.5±27.3 | 79.2±28.3 | ns |
| Night SDNN, ms (mean±SD) | 102.2±33.4 | 76.9±26.5 | <0.0001 |
| Δ[D/N] SDNN, ms (mean±SD) | -21.7±32.9 | 2.3±20.5 | <0.0001 |
| Day r-MSSD, ms (mean±SD) | 30.0±37.1 | 26.7±12.4 | ns |
| Night r-MSSD, ms (mean±SD) | 48.5±34.1 | 35.4±18.4 | <0.05 |
| Δ[D/N] r-MSSD, ms (mean±SD) | - 18.5±17.8 | -8.7±12.7 | <0.001 |
| Day SDNN Index, ms (mean±SD) | 41.8±22.3 | 40.8±15.4 | ns |
| Night SDNN Index, ms (mean±SD) | 78.3±33.8 | 52.9±22.8 | <0.0001 |
| Δ[D/N] SDNN Index, ms (mean±SD) | - 36.5±27.7 | - 12.1±14.7 | <0.0001 |
| Day SDANN, ms (mean±SD) | 65.6±23.6 | 64.4±27.5 | ns |
| Night SDANN, ms (mean±SD) | 56.6±16.8 | 48.8±16.1 | <0.05 |
| Δ[D/N] SDANN, ms (mean±SD) | 5.7±38.3 | 16.5±32.4 | ns |
| Day mean HR, bpm (mean±SD) | 80.1±11.8 | 79.1±11.5 | ns |
| Night mean HR, bpm (mean±SD) | 64.9±8.7 | 67.7±11.8 | ns |
| Δ[D/N] mean HR, bpm (mean±SD) | 15.2±67.1 | 11.4±46.1 | <0.05 |
| Note: HR, heart rate; SDNN Index, mean of the standard deviations of all intervals for all the consecutive 5-minute segments; SDNN, standard deviation of RR intervals; r-MSSD, square root of the mean of the sum of the squares of differences between adjacent normal RR intervals; SDANN, standard deviation of the mean of the consecutive 5-minute segments of RR intervals; Δ[D/N], the differences between day-time and night-time values for the same variables (mean HR, SDNN Index, SDNN, r-MSSD, SDANN). | | | |

There were no differences in clinical characteristics between patients with and without OSAS, in particular neither age nor BMI were helpful in separating the two populations. Mean night SDNN, mean night r-MSSD, mean night SDNN Index, and night SDANN were significantly higher in patients with OSAS, as well as absolute values of Δ[D/N] mean HR, Δ[D/N] SDNN, Δ[D/N] SDNN Index, and Δ[D/N] r-MSSD.

ROC curves (continuous data) were built for each HRV variable; seven of them were able to separate OSAS+ from OSAS- status with statistical significance (p values ranging from 0.03 to < 0.0001; see Table 2).

**TABLE 2.**

| Time-domain HRV Variables Significantly Associated with OSAS by Receiver Operating Characteristic (ROC) Curve Analysis. | | |
|---|---|---|
| Variable | W | P |
| Δ[D/N] SDNN Index | 0.807 | <0.0001 |
| Night SDNN Index | 0.748 | <0.0001 |
| Δ[D/N] SDNN | 0.719 | 0.0007 |
| Night SDNN | 0.709 | 0.0004 |
| Δ[D/N] r-MSSD | 0.692 | 0.002 |
| Night r-MSSD | 0.664 | 0.01 |
| Δ[D/N] mean HR | 0.638 | 0.03 |

Once the seven variables were classified according to their W values, the absolute differences between the day and the night values of the SDNN Index (Δ[D/N] SDNN Index) appeared as the most powerful separator (p<0.0001), followed by the night SDNN Index, the Δ[D/N] SDNN, the night SDNN, the Δ[D/N] r-MSSD, and the night r-MSSD; for each of these variables, the absolute differences between the day and the night values were more potent predictors than the night values by themselves. Finally, the difference between night and day mean heart rate was the last significant variable. None of the time domain values calculated over the day period did reach a statistically significant level.

For each continuous variable, the thresholds giving a chosen sensitivity or specificity were calculated (Table 3).

**TABLE 3.**

| Threshold Values of Continuous HRV Variables for Given Sensitivities and Specificifies | | | | | | |
|---|---|---|---|---|---|---|
| | Sensitivity Threshold | | | Specificity Threshold | | |
| | 70% | 80% | 90% | 70% | 80% | 90% |
| Δ[D/N] SDNN Index (ms) | -19 | -15 | -10 | -17 | -24 | -43 |
| Night SDNN Index (ms) | 58 | 50 | 43 | 63 | 73 | 81 |
| Δ[D/N] SDNN (ms) | -5 | 9 | 18 | -8 | -17 | -26 |
| Night SDNN (ms) | 80 | 75 | 67 | 94 | 97 | 116 |
| Δ[D/N] r-MSSD (ms) | -8 | -7 | -4 | -13 | -21 | -27 |
| Night r-MSSD (ms) | 31 | 27 | 23 | 39 | 50 | 62 |
| Δ[D/N] mean HR (bpm) | 9 | 8 | 6 | 13 | 18 | 22 |

For the most powerful variable, Δ[D/N] SDNN Index, the 90% sensitivity threshold was -10 ms while the 90% specificity threshold reached -43 ms.

The highest separation power was obtained using the CART methodology for the most powerful variables to derive a threshold value with an optimal sensitivity or specificity (see Table 4).

**TABLE 4.**

| Threshold Values of HRV Variables using the Classification and Regression Tree (CART) Methodology | | | | | | |
|---|---|---|---|---|---|---|
| | | | | | Simultaneous 95% CI | |
| | Threshold value | Gini Index | Sensitivity % | Specificity % | Sensitivity % | Specificity % |
| △[D/N] SDNN Index (ms) | -11.1 | 0.132 | 89.7 | 61.5 | [73.7;97.7] | [45.1; 76.3] |
| △[D/N] SDNN (ms) | -34.1 | 0.082 | 30.8 | 98.1 | [15.5;49.8] | [86.4;100] |
| △[D/N] r-MSSD (ms) | -2.5 | 0.060 | 89.7 | 42.3 | [73.7;97.7] | [27.1;58.7] |
| Note: Sens: sensitivity, Spe: specificity, | | | | | | |

We thus recognized Δ[D/N] SDNN Index as an efficient tool for the detection of OSAS and Δ[D/N] SDNN as an efficient parameter to exclude OSAS. For the Δ[D/N] SDNN Index variable, the sensitivity reached 89.7% (95% CI [73.7; 97.7]) with a threshold value of -11.1; and for the Δ[D/N] SDNN variable, the specificity reached 98.1% (95% CI [86.4;100]) with a threshold value of -34.1.

A separate simple logistic regression analysis of diseased versus non-diseased status was performed for each of the covariates to verify the ROC curve analysis and to select a reasonable subset of explanatory variables for further investigation. Maximum likelihood estimates were obtained for each of the important explanatory covariates (Table 5).

**TABLE 5.**

| Separate Simple and Multiple Logistic Regression Analyses | | | | |
|---|---|---|---|---|
| Simple logistic regression analysis Variables | -Log Likelihood | | ChiSquare | Prob>ChiSq |
| Maximum likelihood estimates computed for each variable individually | | | | |
| Δ[D/N] SDNN Index (ms) | 13.72 | | 27.45 | <0.0001 |
| Night SDNN Index (ms) | 8.56 | | 17.12 | <0.0001 |
| Δ[D/N] SDNN (ms) | 8.54 | | 17.09 | <0.0001 |
| Night SDNN (ms) | 7.50 | | 15.01 | 0.0001 |
| Δ[D/N] r-MSSD (ms) | 4.44 | | 8.89 | <0.005 |
| Night r-MSSD (ms) | 3.14 | | 6.28 | <0.02 |
| Age | 0.10 | | 0.21 | 0.649 |

| Multiple logistic regression analysis Term | Estimate | SE | ChiSquare | Prob>ChiSq |
|---|---|---|---|---|
| Likelihood ratio test computed for the whole model (converged by gradient) | | | | |
| Intercept | -3.2205 | 1.6976 | 3.5989 | 0.0578 |
| Δ[D/N] SDNN Index (ms) | -1.3642 | 0.3875 | 12.3944 | 0.0004 |
| Δ[D/N] SDNN (ms) | 0.0737 | 0.1573 | 0.2193 | 0.6395 |
| Δ[D/N] r-MSSD (ms) | 0.7935 | 0.3564 | 4.9584 | 0.0260 |
| Age | 0.3042 | 0.2628 | 1.3401 | 0.2470 |
| BMI | 0.3026 | 0.3569 | 1.3210 | 0.3047 |
| Men/Women | -0.5929 | 0.6408 | 0.8562 | 0.3548 |

These results confirmed the ROC analysis except for the Δ[D/N] mean HR.

With the use of a multiple logistic regression analysis, only two time domain HRV variables appeared to be independently and significantly associated with diseased status. After adjustment for the other variables, Δ[D/N] SDNN Index (p<0.001) and Δ[D/N] r-MSSD (p<0.01) remained significant predictors of OSAS (adjusted odds ratio, 8.22 and 2.86, respectively; confidence intervals [3.16-21.4] and [1.21-6.75], respectively).

In Group 2, neither BMI (28.7±9.8) nor age (52.7±12) were significantly different from those of Group 1. Out of the 52 patients, 24 (46%) were diagnosed as OSAS+ using complete polysomnography. The repartition of patients according to associated disease status (diabetes, hypertension, chronic heart failure, ischemic heart disease) was similar to that of Group 1; the threshold value of -11.1 for Δ[D/N] SDNN Index determined a sensitivity of 83% and the threshold value of-34.1 for Δ[D/N] SDNN determined a specificity of 96.5%.

### f. Discussion

Time domain HRV analysis appears as a powerful tool in investigating OSAS and the differences between day and night values of selected variables are more powerful predictors than their night values taken separately.

Spectral analysis of HRV had already been proposed by Shiomi et al. (see Shiomi T et al. Augmented very low frequency component of heart rate variability during obstructive sleep apnea. Sleep. 1996;19:370-377) who used it to evaluate the benefits of the prosthetic mandibular advancement treatment in OSAS patients; this treatment decreased the power of the very low frequencies without other spectral changes. Furthermore, they did not find any significant change in the time domain variables. Conversely, the present invention shows pertinent criteria in time-domain variables allowing OSAS diagnosis, by creating specific new variables with enhanced discriminant capabilities. These new variables were calculated as the intervals between the lowest and the highest boundary values of three variables, benefiting from an increased contrast between day and night HRV values, particularly Δ[D/N] SDNN Index, and Δ[D/N]r-MSSD.

According to the present invention, new variables are found such as to obtain a high sensitivity (89.7%) and a high specificity as well (98.1%) in the diagnosis of OSAS by chosing appropriate thresholds. The clinical use of these criteria should depend both on the population of interest and on the purpose of the investigator who may want to point towards a greater sensitivity or a greater specificity. A large sensitivity would allow exclusion of the threat of OSAS by using a single Holter recording in patients at risk, as cardiac patients with ponderal excess, while a high specificity would help to increase the probability of the disease if performed as a pre-test in polysomnography centers.

The invention thus offers the possibility of chosing, for each of the three variables used, a given sensitivity and a given specificity by selecting a proper threshold value. Tables 3 and 4 above give some reference values corresponding to specific sensitivities and specificities, on the basis of which a man skilled in the art can vary the threshold value(s) to modify sensitivity and specificity.

From a technical point of view, to be consistent in the comparison between our recordings, we selected the same diurnal and nocturnal periods of HRV analysis. A change in this methodological choice could eventually lead in different results. The threshold for the reference diagnosis of OSAS was selected as 10 events per hour, the more commonly used threshold. Importantly, patients bearing 30 or more events per hour, are identified using the same HRV threshold value with a sensitivity of 96% and a specificity of 99.7%, values much higher than for patients bearing 10 or more events per hour. Such patients are certainly candidates for a specific long-term therapy as nocturnal nasal CPAP.

In summary, time domain analysis of heart rate variability, used as the only criteria, could thus represent an efficient tool in OSAS diagnosis with a sensitivity of 90%. The ease of use and interpretation is also of interest due to the high prevalence of the disease in a general population and the need for repeated control.

### EXAMPLE II

### a).Study group.

The population under study consisted in 124 patients, (98 males, 26 females) with a mean (±SD) age of 53.8±11.2 years (range 22 to 78), who were referred to our university hospital for a polysomnography recording based on clinically suspected OSAS. Exclusion criteria were permanent or paroxysmal atrial fibrillation, diabetes mellitus, autonomic neuropathy, Shy-Drager syndrome, and permanent ventricular or atrial pacing. In all patients the 24-hour ECG Holter monitoring started 6 hours before the beginning of the polysomnogram recording and was continued so as to obtain a standard 24-hour duration ECG recording. These prospective investigations at the pulmonary and at the cardiac level were performed by two complementary clinical teams, each of them blinded to the results of the other; 18 patients were recorded a second time in the same conditions after a 3-month treatment with continuous positive airway pressure (CPAP).

### b). Sleep study and polysomnography scoring.

OSAS was diagnozed on the basis of clinical criteria and on the polysomnography performed following the recommendations of the American Sleep Disorders Association (see Thorpy MJ, and the Diagnostic Classification Steering Committee. The International Classification of Sleep Disorders : Diagnostic and Coding Manuel. Rochester, Minn: American Sleep Disorders Association; 1990:52-58). The presence and stages of sleep were monitored using two pairs of electroencephalographic leads (C₄-A₁, O₂-A₁), two pairs of electrooculographic leads and chin electromyographic leads. Airflow was measured by an oronasal thermocoupler. Respiratory efforts were monitored using inductive plethysmography, with transducers placed around the chest and the abdomen. Arterial oxygen saturation was recorded continuously by pulse oximetry (Criticare Systems Inc., USA), during the whole night period.

The polysomnogram was scored manually according to standard criteria (see Rechtschaffen A, Kales A eds. A manual of standardized terminology, techniques and scoring systems for sleep stages of human subjects. Los Angeles: BIS/BRI UCLA; see also : C. Guilleminault et al. Sleep and breathing. In: Guilleminault C, ed. Sleeping and Waking Disorders: Indications and Techniques. Stoneham, UK: Butterworth Publishers; 1982:155-182). Apnea was defined as the absence of airflow for more than 10 seconds in the presence of persistent respiratory efforts. Hypopnea was defined as the association of a reduction of 50% or more of the amplitude of respiratory efforts during at least 10 seconds, associated to a fall in SaO₂ of at least 4%. The apnea/hypopnea index (AHI) was defined as the number of episodes of apnea and hypopnea per hour of sleep and reflected the respiratory index disorder; the threshold to identify OSAS was chosen as an AHI equal or beyond 10. Apnea duration, as well as mean and minimal SaO₂ were determined for each episode of apnea. Total sleep time (TST), number and duration of REM periods, number and duration of arousals, were evaluated. Arterial desaturation was quantitated as the total time with SaO₂ below 90%.

### c). 24-hour ECG Holter monitoring and heart rate variability analysis.

Holter tapes were analyzed on a Duolter Novacor System (Rueil Malmaison, France), equipped with the heart rate variability module. To perform the analysis, each QRS complex was validated and the length between each QRS (RR interval) calculated. Only normal to normal beats were considered for analysis. Mean duration of Holter recording reached 22±2 hours.

Time-domain measurements were performed (see Task Force of the European Society of Cardiology and the North American Society of Pacing and Electrophysiology. Heart rate variability: standards of measurements, physiological interpretation, and clinical use. Circulation. 1996;93:1043-1065) over three different periods: the full recording duration, and on a day and a night period separately, in order to obtain the derived parameters which, as described above, represent the contrast between day and night values. The r-MSSD (root mean square of successive differences between adjacent normal RR intervals), pNN50 (percent of adjacent normal RR intervals greater than 50 msec), SDNN (Standard deviation of all normal RR intervals), SDANN (standard deviation of the mean of all consecutive 5-minute segments of normal RR intervals), and SDNN Index (mean of the standard deviations of the consecutive 5-minute segments) were computed; the derived day/night differences in these parameters was then calculated: Δ[D/N] r-MSSD, Δ[D/N] SDANN, Δ[D/N] SDNN , Δ[D/N] SDNN Index. A classical Fourier transform analysis of the RR intervals was then performed only over the full recording period. However, it is well established that this analysis does not allow a precise quantification over the very low frequency level.

To better identify the very-low frequency oscillations, we performed a power spectral analysis of the interbeat interval increment (PS3I) as a function of the inverse of the increment lengh. The spectral data were then smoothed by a sliding average over fifty consecutive values. We calculated the absolute value of the power spectral density of that very low frequency band (0.01 to 0.05 beat⁻¹, VLFIpsd), as well as the relative importance of this component (%VLFI) over the total power spectral density (0.01 to 0.5 beat⁻¹, TIpsd). In the same manner, the power spectral density of the low frequency band (0.05 to 0.15 beat-1, LFIpsd), and of the high-frequency band (0.15 to 0.40 beat⁻¹, HFIpsd) as well as their expression in % of the total power spectral density (respectively %LFI, %HFI) were also computed.

### d). Statistical analysis.

Data were analyzed using Statview and JMP (SAS Institute® ) softwares. Differences were considered as significant when p <0.05. Values were expressed as mean and 95% standard deviations (mean±SD). Statistical analysis were performed to evaluate the ability of the variables to discriminate between non-diseased and diseased status. Thus, the dependent variable was the diseased status (OSAS +/-). The independent variables analyzed were age, gender, body mass index (BMI), frequency-domain parameters of the PS3I (VLFI, LFI, and HFI components), their expression as a percentage of the total power spectral density (%VLFI, %LFI, %HFI, respectively), as well as the three time-domain parameters that were significantly associated to the disease status as disclosed above.

ROC curve analysis was used (see Hanley JA, McNeil BJ. : The meaning and use of the area under receiver operating characteristic (ROC) curve. Radiology. 1982; 143:29-36) with the areas under the curves represented by the letter W. A W value of 0.5 means that the distribution of the variables are similar in both populations; conversely, a W value of 1.0 means that the distributions of the variables of two populations variable distributions do not overlap at all.

Logistic regression models were used to further analyze the data via simple logistic regression of the diseased status versus each of the covariates, to confirm the previously performed ROC curves analysis. Multiple logistic regression analysis was then used to evaluate the strength of the association of each variable with the diseased status, after adjustment for the other variables. Odds ratios were calculated individually for the most significant independent variables

Finally, a linear correlation was established between, on one hand, the AHI and, on the other hand, the variables significantly and independently associated with the diseased status.

### e). RESULTS

The diagnosis of OSAS was established in 54 (43.5%) of the 124 patients, using polysomnography recording. The clinical variables of all patients are summarized in Table 6 below. There were no differences in clinical characteristics between patients with and without OSAS, in particular neither age nor BMI were helpful in separating the two populations.

**TABLE 6.**

| Clinical Characteristics | | | |
|---|---|---|---|
| Clinical characteristics | OSAS+ | OSAS - | p |
| | (n = 54) | (n =70) | |
| Age, yr (mean±SD) | 55.8±11.2 | 54.9±10.7 | ns |
| Male, n (%) | 42(78) | 55(79) | ns |
| BMI, kg/m² (mean±SD) | 28.9±9.2 | 29.9±9.9 | ns |
| Hypertension, n (%) | 20(37) | 15(21) | ns |
| Note: BMI, body mass index; OSAS+, patients with obstructive sleep apnea syndrome; OSAS-, patients whitout obstructive sleep apnea syndrome. | | | |

Mean±SD VLFI power spectral density was significantly higher in patients with OSAS (0.18±0.19 vs 0.06±0.06 ms².beat⁻¹; p<0.0001) as well as the mean±SD %VLFI (5.84±3.60 vs 2.81±2.16 ms².beat⁻¹; p<0.0001). Neither any other frequency-domain variable nor standard time-domain parameters of HRV calculated over the 24-hour recordings showed any significant alteration in OSAS patients. However, as shown in Table 7 below, mean Δ[D/N] SDNN Index, Δ[D/N] r-MSSD and ΔD/N] SDNN were found significantly higher in OSAS patients :

**TABLE 7.**

| Time-Domain and Frequency-Domain of the Heart Rate Variability (HRV) Characteristics. | | | |
|---|---|---|---|
| Time-Domain HRV | OSAS + (n=54) mean±SD | OSAS - (n=70) mean±SD | p |
| HR (bpm) | 73.19±9.37 | 73.39±11.73 | ns |
| pNN50 (%) | 10.79±8.±88 | 9.70±9.41 | ns |
| r-MSSD(ms) | 32.60±12.38 | 31.88±14.48 | ns |
| SDNN Index (ms) | 56.86±20.57 | 47.11±18.63 | ns |
| SDNN (ms) | 117.59±40.81 | 108.13±39.49 | ns |
| SDANN (ms) | 98.69±36.29 | 91.55±36.06 | ns |
| Δ[D/N] r-MSSD | -18.59±15.02 | -10.38±13.1 | <0.001 |
| Δ[D/N] SDNN Index | -37.69±25.94 | -12.80±14.33 | <0.0001 |
| Δ[D/N] SDNN | -18.34±34.49 | 2.50±20.72 | <0.0001 |

| Frequency-Domain HRV (PS3I) | | | |
|---|---|---|---|
| TI psd (ms².beat⁻¹) | 3.52±2.91 | 2.97±2.73 | ns |
| VLFIpsd (ms².beat⁻¹) | 0.18±0.19 | 0.06±0.06 | <0.0001 |
| LFIpsd (ms².beat⁻¹) | 0.35±0.29 | 0.28±0.33 | ns |
| HFIpsd (ms².beat⁻¹) | 2.20±2.15 | 1.91±2.09 | ns |
| %VLFI | 5.84±3.60 | 2.81±2.16 | <0.0001 |
| %LFI | 10.27±4.93 | 10.06±7.74 | ns |
| %HFI | 58.88±13.03 | 59.76±12.01 | ns |
| Note: OSAS+, patients with obstructive sleep apnea syndrome; OSAS-, patients whitout obstructive sleep apnea syndrome;HR, heart rate; pNN50, percent of adjacent normal RR intervals greater than 50 msec; r-MSSD, square root of the mean of the sum of the squares of differences between adjacent normal RR intervals; SDNN Index, mean of the standard deviations of all intervals for all the consecutive 5-minute segments; SDNN, standard deviation of RR intervals; SDANN, standard deviation of the mean of the consecutive 5-minute segments of RR intervals; Δ[D/N], differences between day-time and night-time values for the same variables (r-MSSD, SDNN Index, SDNN); Total PSD, total power spectral density; VLFI psd, power spectral density of the very low frequency components; LFI psd, power spectral density of the low frequency components; HFI psd, power spectral density of the high frequency components; %, ratio of the power spectral density of the frequency band (Interbeat interval increment) on the total power spectral density | | | |

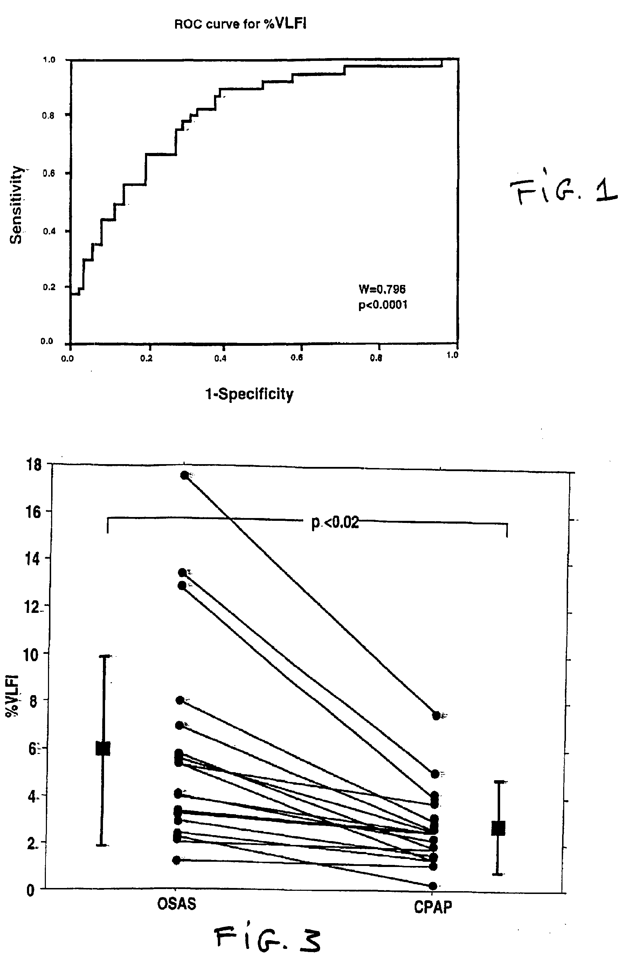

ROC curves (continuous data) were built for each frequency-domain HRV variable. Only two of them (VLFIpsd and %VLFI) were able to separate OSAS+ from OSAS- status with statistical significance (p< 0.0001). Once the variables were classified according to their W values, Δ[D/N] SDNN Index appeared as the most powerful separator (W= 0.813, p<0.0001; see Table 8 below), followed by the %VLFI (W=0.796, p<0.0001; Table 8 and Fig.1) and the VLFI component, expressed as an absolute power spectral density (W=0.788, p<0.0001). In accordance with the first part of the invention, Δ[D/N] SDNN and Δ[D/N] r-MSSD were also significant predictors of OSAS (W=0.676, p<0.002 and W=0.667, p<0.002, respectively; Table 8).

**TABLE 8.**

| HRV Variables Significantly Associated with OSAS by Receiver Operating Characteristic (ROC) Curve Analysis | | |
|---|---|---|
| Variable | W | P |
| Δ[D/N] SDNN Index | 0.813 | <0.0001 |
| %VLFI | 0.796 | <0.0001 |
| VLFIpsd | 0.788 | <0.0001 |
| Δ[D/N] SDNN | 0.676 | <0.002 |
| Δ[D/N] r-MSSD | 0.667 | <0.002 |

The stability of the interbeat interval increment determined for a given patient the presence of a characteristic very-low frequency (VLFI) peak (mean±SD frequency 0.03257± 0.009 beat⁻¹; range 0.01709-0.04954 beat⁻¹). Such a peak, corresponding to the breathing pattern disorder, was visually identified in 51 out of the 54 OSAS patients.

Using multiple logistic regression analysis, only 2 time-domain and 1 frequency-domain HRV variables appeared to be independently and significantly associated with diseased status (see Table 9 below). After adjustment for the other variables, Δ[D/N] SDNN Index, Δ[D/N] r-MSSD, and %VLFI remained significant predictors of OSAS (adjusted ORs, 8.2, 2.9 and 8.4; respectively ; 95% CI: 3.0 to 21.5 , 1.21 to 6.7, and 3.36 to 19.5, respectively).

**TABLE 9.**

| Simple and Multiple Logistic Regression Analyses | | | | |
|---|---|---|---|---|
| Simple logistic regression analysis | -Log Likelihood | | | |
| Variables | | | ChiSquare | Prob>ChiS q |
| Maximum likelihood estimates computed for each variable individually | | | | |
| Δ[D/N] r-MSSD | 5.21 | | 10.43 | <0.001 |
| Δ[D/N] SDNN Index | 20.55 | | 41.11 | <0.0001 |
| Δ[D/N] SDNN | 8.15 | | 16.31 | <0.0001 |
| VLFIpsd | 16.87 | | 33.75 | <0.0001 |
| %VLFI | 15.48 | | 30.97 | <0.0001 |

| Multiple logistic regression analysis | | | | |
|---|---|---|---|---|
| Term | Estimate | SE | ChiSquare | Prob>ChiS q |
| Likelihood ratio test computed for the whole model (converged by gradient) | | | | |
| Intercept | -2.650 | 0.531 | 24.92 | <0.0001 |
| Δ[D/N] r-MSSD | 0.066 | 0.033 | 3.85 | 0.0497 |
| Δ[D/N] SDNN Index | -0.082 | 0.028 | 8.67 | 0.0032 |
| Δ[D/N] SDNN | 0.020 | 0.013 | 2.49 | 0.1054 |
| VLFI psd | 6.057 | 4.901 | 1.53 | 0.2166 |
| %VLFI | 0.211 | 0.103 | 4.13 | 0.0421 |

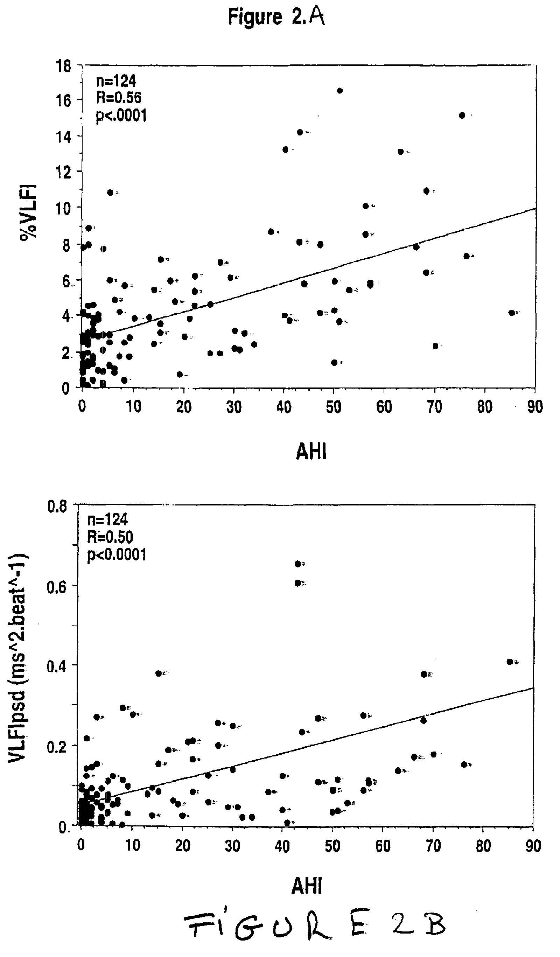

A significant, however weak, positive correlation (Fig. 2) was found between, the AHI and the two continuous previously described frequency-domain variables of the HIII (%VLFI, r=0.56, p<0.001; VLFI, r=0.50, p<0.01). Furthermore, a significant negative correlation was found between the RDI and the three time-domain HRV variables significantly predictive of OSAS, Δ[D/N] SDNN Index, Δ[D/N] r-MSSD and Δ[D/N] SDNN (r=0.73, p<0.0001; r=0.52, p<0.001; r=0.47, p<0.0001, respectively).

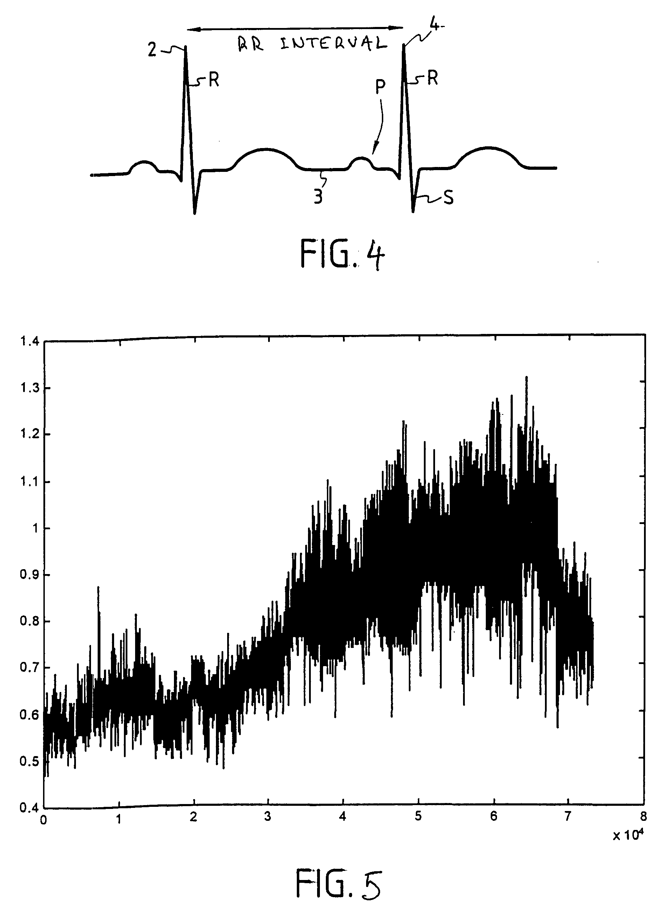

In the 18 patients with OSAS who underwent a second Holter monitoring during a control polysomnogram after a 3 month treatment with continuous positive airway pressure (CPAP), the apnea/hypopnea index decreased from 50.6±13 to 2.2±2.5 event per hour. CPAP treatment determined a significant decrease (Fig.3) in %VLFI component (2.1±1.3 vs 4.8±3.1%; p<0.03) and a reduction in Δ[D/N] SDNN Index, Δ[D/N] r-MSSD (-22.8±21.7 vs -48.3± 31.2 and -15.8±10.6 vs -23.5±12.3; p<0.03 and p<0.05 respectively).

### F). DISCUSSION

Repeated episodes of sleep apnea determined a characteristic and identifiable increase in the very-low frequency component of heart rate variability, when calculated from the interbeat interval increment. In our population, the presence of such a increased very-low frequency spectral power density was highly predictive of a sleep-related breathing disorder and, as an independant predictor of sleep-related disease, is thus a potential powerful ambulatory identifier to screen patients with a clinical suspicion of OSAS.

After further analysis, we found spectral analysis of the interbeat interval increment as a powerful tool to be evaluated.

The diagnostic yield of the very low frequency-domain (frequencies up to 0,04 Hz) parameters was systematically tested by the inventors on a general population of patients suspected of OSAS.

Unlike Δ[D/N] SDNN Index and Δ[D/N] r-MSSD, the diagnosis power of the new variable, %VLFI, was not dependent from a contrast between its day and night values. This is of interest since sleep periods are sometimes difficult to authentify. It is thus difficult to identify on recordings which parts should be selected from the whole recording, and it is also very difficult to conduct recordings only during the night or the day, in particular for a large population. Furthermore, this new parameter %VLFI allows an easier use of a predictor algorithm, since the pertinent data is automatically summarized by a simple number.

The %VLFI, or the visual indication of the very low frequency peak of the power spectral density, can be used alone or in combination with any of the parameters already disclosed above, namely :
* the difference between night and day square root of the mean of the sum of the squares of differences between adjacent normal RR intervals (Δ[D/N] r-MSSD),
* the difference between night and day mean of the standard deviation of all normal-normal intervals for all consecutive at least 5 minutes segments of the recording (Δ[D/N] SDNN index),
* the difference between night and day standard deviation of the normal-normal intervals (Δ[D/N] SDNN).

The %VLFI and these parameters form a group of independant determining variables.

The accuracy of HRV variables in OSAS detection is further reinforced by the linear relationship between VLFI and AHI. Although weak, this correlation is statistically significant and reinforces the security over the diagnosis of OSAS since, the lowest the %VLFI, the lowest the severity of an associated disease. Thus, the relationship between AHI and VLFI opens the possibility to use this indice as a powerful indicator of treatment efficacy that could ease sleep laboratories overload.

Altered respiratory patterns are not restricted to patients with OSAS, but also occur in mild to severe chronic heart failure. Recent polysomnographic studies reported a high prevalence of sleep-related breathing disorders in stable chronic heart failure patients (see Javaheri S et al. Sleep apnea in 81 ambulatory male patients with stable heart failure: types and their prevalences, consequences, and presentations. Circulation. 1998;97:2154-59). In the invention, slow cardiorespiratory oscillations produced a large increase in HRV and particularly in VLF power. This could justify a pre-screening step using the present algorithm to detect contamination of the VLF band with discrete peaks related to periodic-breathing disorders in those populations.

The invention thus concerns the detection of sleep disorders with the help of the %VLFI value or with the presence of a peak in the very low frequency range of the power spectral density.

Complete polysomnography, as well as continuous nocturnal oxygen saturation monitoring, also carry some inaccuracies: transducer position, failure, and displacement; artefacts due to movements; and alteration of the sleep characteristics because technical environment. Most importlantly, Stradling et al., (New approaches to monitoring sleep-related breathing disorders. Sleep 1996;19:S77-S84) underlined that classical polysomnography could represent a flattened gold standard, as measuring apneas, hypopneas or hypoxemic events was only logging some of the respiratory abnormalities.

The frequency-domain HRV variables obtained from 24 h ECG Holter monitoring were compared to respiratory disturbances Indexes (RDI) assessed by complete polysomnography in 124 consecutive patients (98 males) aged 53.8±11.2 (mean±SD) with clinically suspected OSAS; the predictive accuracy of the power spectral density of the interbeat interval increment (VLFIpsd: 0.01 to 0.05 beat⁻¹) and its percentage over the total power spectral density (%VLFI) were analyzed. These data were also compared to established time-domain HRV variables. OSAS was present in 54 (43.5%) of patients according to standard criteria (RDI=10 events /h). Using ROC curves analysis, the three most powerful predictor variables were, Δ[D/N] SDNN Index (W=0.81, p<0.0001), %VLFI (W=0.80, p<0.0001), and VLFIpsd (W=0.79, p<0.0001) respectively. Using a multiple logistic regression analysis, three variables appeared to be independently and significantly associated with diseased status: %VLFI, Δ[D/N] SDNN Index, and Δ[D/N] r-MSSD (adjusted OR, 8.4, 8.2 and 2.9; 95% CI, 3.36 to 19.5, 3.0 to 21.5, and 1.2 to 6.7, respectively). CPAP treatment determined a significant decrease in these three independant HRV parameters.

According to the invention, frequency-domain analysis of heart rate variability using power spectral power of the interbeat interval increment represents an efficient tool in screening for OSAS. Its ease of use and of interpretation is of interest considering the high prevalence of the sleep-related breathing disorders in a general middle-aged population. Furthermore, it could become an intersting tool for monitoring treatment efficacy on an ambulatory basis.

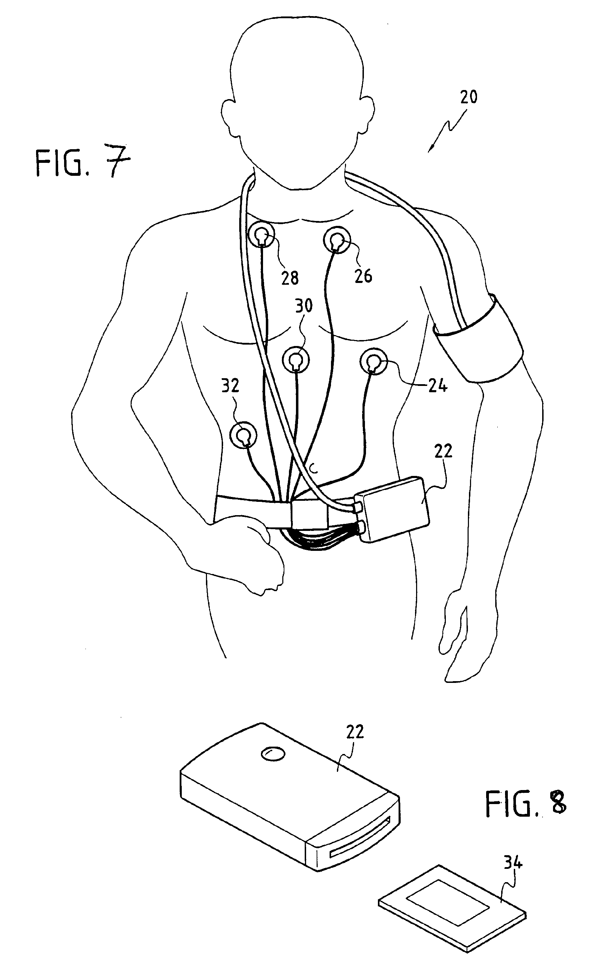

Figure 6 represents steps for obtaining an RR signal from a digital electrocardiographic (ECG) recording. Such an ECG recording is shown on figure 4. The first two steps (6-1 and 6-2) are performed with a standard device of the "Holter" type.

Each QRS complex is classified as normal, ventricular or supraventricular. Normal and supraventricular QRS complexes show P waves just before the R peak, whereas abnormal (ventricular) complexes do not. Ventricular QRS are actually beats which spontaneously originate from the heart "ventricules". They are generally larger than the normal ones. They can be identified by their width which is larger than the width of the other complexes.

The RR intervals associated with supraventricular complexes are different from the preceeding RR intervals (for example averaged over a certain number of preceeding RR intervals, for example the 8 or 10 preceeding RR intervals) of more than 20%. Supraventricular complexes can thus be easily detected and eliminated.

One thus creates a table or a database comprising the QRS (identified by its position in the list of QRS) and the type of QRS for each QRS.

The normal complexes are selected from this table or database (step 6-3). An RR signal, or a signal of the interbeat interval increments, can then be derived therefrom (6-4) by calculating the difference between two successive R peaks.

Figure 4 is an example of part of an ECG, with two QRS complexes each having a peak 2, 4.

An RR recording obtained with a Holter device is shown on figure 5. It contains more than 70 000 RR intervals recorded over 24 hours. The figure shows the RR intensity versus peak index (or order) in the series of RR peaks.

Figure 7 shows how a recording is made on a patient 20 provided with a "Holter" device 22, with electrodes 24,26,28,30,32 disposed on the patient's body. The Holter device is for example a Novacor product (Novacor, 4, passage Saint Antoine, 92508, Rueil malmaison - France), for example the Unolter device or the Duolter device. The recording device can also be of any other type known in the art for recording electrocardiograms.

The recording device records the ECG data during one night (which means: comprising the patient's sleep period or for example from one of the following nighttimes : 22h00 or 23h00 or 24h00 to one of the following daytimes: 6.00 am or 7.00 am or 8.00 am) or one day (for example from one of the following daytimes : 8:00 am or 9:00 am or 10:00 am or 12:00 am, to any of the following daytimes or evening times : 5:00 pm or 6:00 pm or 7:00 pm or 8:00 pm or 9:0pm or 10:00 pm) or during day and night, for example during 24 hours. The recording duration actually depends on which type of analysis according to the invention is performed: for an analysis based on one or more of parameters (Δ[D/N] r- MSSD), (Δ[D/N] SDNN index), (Δ[D/N] SDNN), recordings require night recordings and day recordings, whereas recordings for %VLFI tests or VLFI spectral peak analysis can be 24 hours recordings or recordings over a period longer than one night (for example during between 14 hours and 18 or 24 hours) thus eliminating the need to precisely identify night and day periods.

As illustrated on figure 8 the data are recorded on a memory card 34 of the flash type, for example with a capacity of 20 Mbytes. Recordings can also be made on tapes, with another type of Holter apparatus.

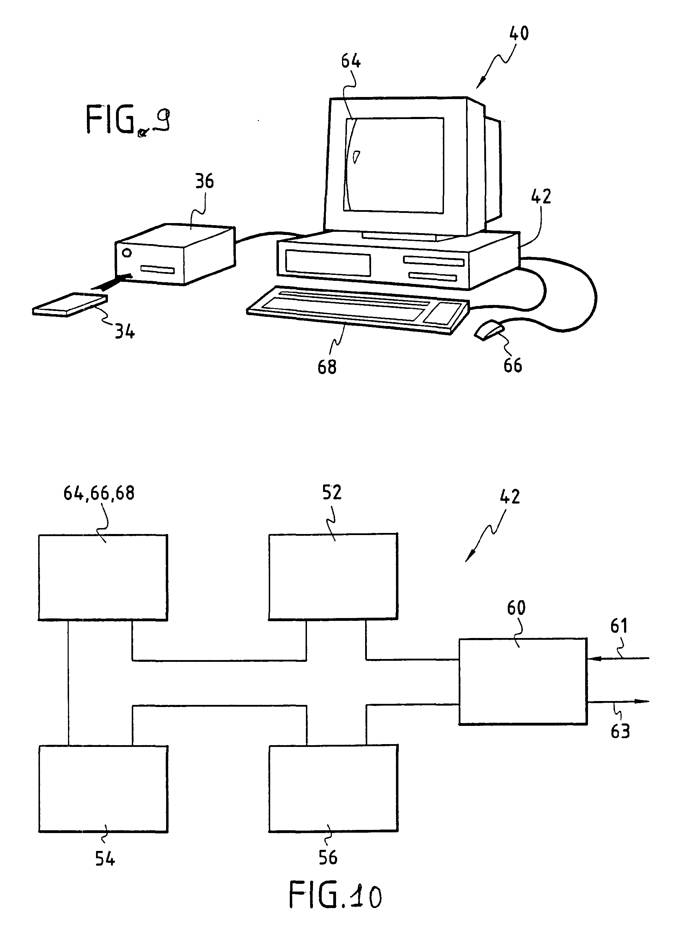

Figure 9 shows a device 40 for analyzing data or signals recorded on a memory means 34, for example a data card. Memory means 34 is introduced into a reader 36, for example a PCMCIA reader, and the data are treated accordingly, device 40 being programmed for performing the necessary treatment steps.

Such a device comprises a microcomputer 42 to which data read by reader 36 are transmitted via connection 41.

Microcomputer 42 comprises (figure 10) a microprocessor 52, RAM memories 54 (for storing data), ROM memories 56 (for storing program instructions).

A card 60 for data acquisition adapts the data sent by reader to the the format specificied by the microcomputer. The various elements of the computer are connected through a bus 62.

An operator can interactively communicate with the computer through peripheral devices (screen or display device 64, mouse 66, keyboard 68). Display means 64 displays the RR intervals and/or the power spectral density of the interbeat interval increment and/or any calculated value of any parameter. The operator can also initialize a threshold value for any of the (Δ[D/N] r- MSSD), (Δ[D/N] SDNN index), (Δ[D/N] SDNN) or %VLFI variables. With help of icons or of a specific menu on screen 64, the operator can also vary any of these threshold values as already explained above, in order to vary the corresponding sensitivities and specificities.

Sensitivity can be selected in the range of 80%-90% (for example : 85%) and specificity in the range of 50%-100% (for example : 60%).

The % VLFI parameter threshold value is preferably comprised between 1,8% and 2,4%. For a value of 2%, the sensitivity is about 90% and the specificity is about 68%.

The Δ[D/N] SDNN Index parameter threshold value is preferably comprised between -10ms and -15ms, with a corresponding sensitivity between 80% and 90% (see Table 3). The specificity can be deduced from ROC curves.

The Δ[D/N] r- MSSD parameter threshold value is preferably comprised between -4ms and -7ms, or even between -7ms and -2,5ms, with a corresponding sensitivity between 80% and 50% (see Tables 3 and 4). The specificity can be deduced from ROC curves.

A doctor or a cardiologist can thus see the different curves, totally or in part, with or without magnification, and/or the figures calculated by the computer.

The instructions for performing a data processing according to the invention are loaded in microcomputer 42.

These instructions can be loaded into memory means of the computer 42 from a diskette or a floppy disk or any other memory means readable by a microcomputer (for example: hard disk, ROM memory, DRAM or RAM, optical compact disk, magnetic or optical storage device).

The normal peaks or QRS complexes are first separated from the rest of the ECG, and the peak-to-peak intervals are calculated, thus generating the RR intervals.

In a first method according to the invention, one or more of the following values are calculated: (Δ[D/N] r- MSSD), (Δ[D/N] SDNN index), (Δ[D/N] SDNN).

In another method according to the invention, %VLFI of the power spectral density of the interbeat interval increment is calculated from a recording made over a time period longer than one patient's night, for example 12 or 14 or 16 or 18 or 20 or 24 hours.

In both methods, there is no need for any other recording like polysomnography or oxymetry or respiratory recordings.

Figure 11 is a partial view of the power spectral density of the interbeat interval increment for a normal patient. This curve is displayed on display means 64, for example together with the calculated %VLFI. No peak can be seen or detected in the very low frequency range (for example between 0.01 Hz and 0.04 Hz).

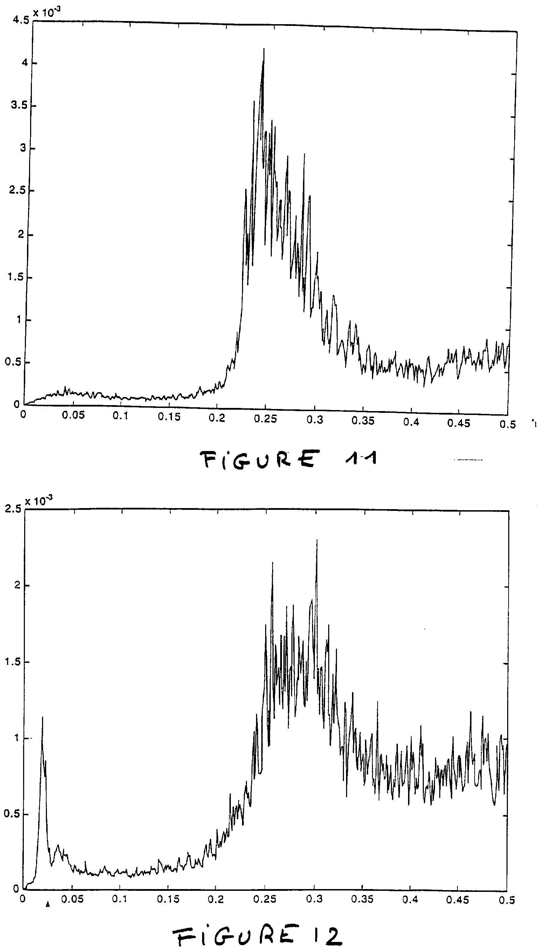

Figure 12 is a partial view of the power spectral density of the interbeat interval increment for a patient showing apnea. A strong peak can be seen in the frequency range between 0.01 Hz and 0.04 Hz.

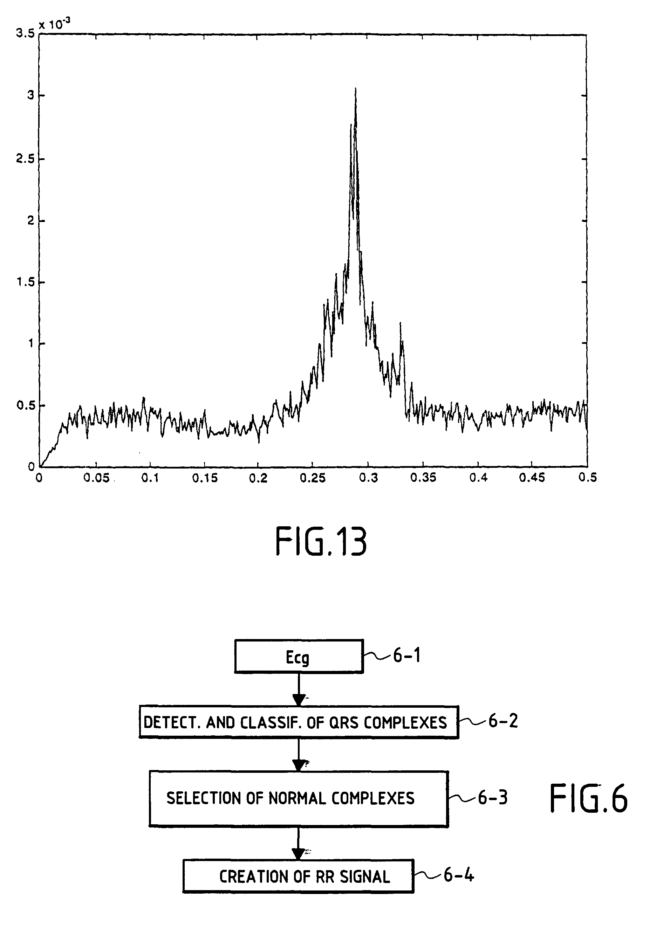

Figure 13 concerns the case of a patient having apnea controlled with a CPAP (Continue Positive Airway Pressure) treatment. A strong reduction of the very low frequency peak is observed.

All these curves of figures 11 - 13 are diplayed on display means 64. The cardiologist can then look at these results and confirm whether or not the patient suffers from apnea.

The same curves can be used for identifying other types of sleep disorders, for instance in the case of mild to severe chronic heart failure. Innsuch a case, the method is performed as disclosed above: an ECG is recorded, normal peaks are selected therefrom, and the spectral density of the interbeat interval increment is calculated and displayed.

## Claims

1. A method for processing an RR intervals signal, derived from an ECG signal, comprising :
- storing RR intervals and calculating the variability of the RR intervals,
- calculating the power spectral density of the interbeat interval increment and exhibiting said portion of the spectrum thus obtained which is comprised below 0.04 Hz, or calculating the relative importance of the component of the spectrum which is comprised below 0.04 Hz over the total power spectral density and comparing said importance with a predetermined threshold value.

2. A method according to claim 1 said threshold value being selected according to a predetermined sensitivity and specificity.

3. A method according to any of claims 1 or 2, said ECG signal being recorded over a time period longer than 10 hours.

4. A method for processing an RR signal, derived from the normal beats of an ECG signal, comprising :
- storing RR intervals,
- calculating at least one of the following parameters:
* difference between night and day square root of the mean of the sum of the squares of differences between adjacent normal RR intervals (Δ[D/N] r-MSSD),
* difference between night and day mean of the standard deviation of all normal-normal intervals for all consecutive at least 5 minutes segments of the recording (Δ[D/N] SDNN index),
* difference between night and daystandard deviation of the normal-normal intervals (Δ[D/N] SDNN),
- comparing said at least one parameter with at least one predetermined threshold value.

5. A method according to claim 4, said at least one threshold value being selected according to a predetermined sensitivity and specificity.

6. A method according to any of claims 4 or 5, only those normal to normal intervals beats being considered for calculating said at least one parameter.

7. A method according to any of claims 1 to 6, the ECG data being first recorded with a Holter device.

8. A device for processing an RR intervals signal, derived from an ECG signal, comprising :
- means for storing RR intervals and the variability of the RR intervals,
- means for calculating the power spectral density of the interbeat interval increment and exhibiting said portion of the spectrum thus obtained which is comprised below 0.04 Hz, and/or for calculating the relative importance of the component of the spectrum which is comprised below 0.04 Hz over the total power spectral density and comparing said importance with a predetermined threshold value.

9. A device according to claim 8, further comprising means for adjusting said predetermined threshold value.

10. A device for processing an RR signal, derived from the normal beats of an ECG signal, comprising :
- means for storing RR intervals,
- means for calculating at least one of the following parameters:
* difference between night and day square root of the mean of the sum of the squares of differences between adjacent normal RR intervals (Δ[D/N] r-MSSD),
* difference between night and day mean of the standard deviation of all normal-normal intervals for all consecutive at least 5 minutes segments of the recording (Δ[D/N] SDNN index),
* difference between night and daystandard deviation of the normal-normal intervals (Δ[D/N] SDNN),
- means for comparing said at least one parameter with at least one predetermined threshold value.

11. A device according to claim 9, further comprising means for adjusting said at least one predetermined threshold value.

12. A device according to any of claims 9 or 10, further comprising one or more Holter devices.
